# EUROPEAN PATENT SPECIFICATION

(11) **EP 2 074 967 B1**
(45) Date of publication and mention of the grant of the patent: **21.06.2017**
(21) Application number: 08171314.1
(22) Date of filing: 11.12.2008
(51) Int. Cl.: A61F 2/958

(54) **A method of retaining a polymeric stent on an expansion member**
Verfahren zum Halten eines polymeren Stents an einem Erweiterungselement
Procédé de retenue d'une endoprothèse polymère sur un élément d'expansion

(30) Priority: 19.12.2007 US 959619
(43) Date of publication of application: 01.07.2009
(73) Proprietor: Cordis Corporation, Miami Lakes, Florida 33014 (US)
(72) Inventor: Dave, Vipul, Hillsborough, NJ 08844 (US); Donovan, Ryan R., Mountain View, CA 94043 (US); Peng, Eileen, Basking Ridge, NJ 07920 (US)
(74) Representative: Prock, Thomas

(56) References cited:
- WO-A-03/092550
- US-A- 6 063 092

## Description

The present invention generally relates to delivery systems for implanting an expandable polymeric stent within a vessel or other passageway of a patient and more particularly to a method for improving the retention of a polymeric stent on a balloon during delivery into the vasculature of a patient.

It is desirable to employ medical devices to improve the patency of anatomical passageways within the human body. For example, stents maintain or restore the patency of an anatomical passageway of a patient and can be implanted without an invasive procedure. Stents can be constructed from metal or polymeric material and generally comprise a lattice structure. In particular, the lattice comprises a series of longitudinal struts each joined together by a curved member in a continuous manner so as to form a cylindrical element. Each cylindrical element is attached to an adjacent cylindrical element by connecting members. The flexibility and strength of the stent can be varied by adding cylindrical elements, connecting members and/or struts. In addition, the thickness and geometry of the stent components can be varied.

Stents are typically constructed from a metallic material that can be crimped to a first low profile shape for passage through the vasculature of a patient. Upon reaching the desired deployment site, the stent is then expanded to a second profile to restore the patency of the vessel. Metallic stents can be constructed from self-expanding super-elastic material or can be balloon expandable and constructed from a plastically deformable material. Once in place, however, a metallic stent remains in the vessel even after the patency of the vessel has been restored. This has several potential drawbacks, for example, the vessel may be damaged by the stent, and the stent may prevent re-intervention at distal locations within the vessel. In order to overcome these limitations, bioabsorbable stents have been developed. These stents are constructed from polymeric materials that are less stressful to surrounding tissue and are resorbable.

For endovascular implantation of a stent into a blood vessel, percutaneous deployment is initiated by an incision into the vascular system of the patient, typically via the femoral or carotid artery. A tubular or sheath portion of an introducer is inserted through the incision and into the artery. According to one method for implantation, there is a central lumen through the introducer that provides a passageway through the patient's skin and artery wall into the interior of the artery. An outwardly tapered hub portion of the introducer remains outside the patient's body to prevent blood from leaking out of the artery along the outside of the sheath. A valve provided on the introducer is manipulated to block blood flow out of the artery through the introducer passageway. A distal end of a guide wire is passed through the introducer passageway and into the patient's vasculature. The guide wire is threaded through the vasculature until the inserted distal end of the guide wire extends just beyond the intended treatment site. The proximal end of the guide wire typically extends outside the introducer for manipulation by the medical practitioner. Once the wire is in place, a catheter having an expansion member such as a balloon near its distal end is fed over the wire until its distal end is located at the treatment site.

A typical balloon catheter comprises an elongate and flexible shaft defining one or more passages or lumens, and an inflatable balloon mounted at one end of the shaft. The balloon is in fluid communication with one of the lumens allowing for inflation of the balloon. The opposite end of the shaft lumen includes a hub for accessing the various lumens of the shaft, for example, providing a means to place the shaft over a guide wire that exits out the distal end of the shaft or places one of the lumens in communication with an inflation source. Some shafts include an access port allowing the wire to enter the shaft just proximal to the distal end of the shaft, exiting at the tip. An expandable medical device such as a stent is placed on the balloon such that inflation of the balloon will expand the stent.

In order to place the stent in the desired location, the balloon catheter is navigated within the vasculature of the patient. The tortuous nature of the vasculature can complicate the delivery of the stent. For example, the stent may contact the inner walls of the vasculature causing it to slide along the surface of the balloon. A metallic stent can be tightly crimped or compressed onto the balloon to prevent slippage. This does not always prevent slippage, however, as most balloons have a relatively smooth surface and over crimping can lead to puncture or damage of the balloon. One attempt at solving this is disclosed in U.S. Patent No. 6,942,681 to Johnson. Johnson discloses a balloon having geometric features that cooperate with the structural elements of the stent. One example shows a balloon having bumps that fit within the lattice of the stent preventing lateral movement of the stent during navigation through the vasculature to the targeted site for deployment.

While a variable geometry balloon such as the one disclosed in Johnson is effective for crimping a metallic stent to a balloon, crimping a polymeric stent is more difficult due to the elastic nature of the material. This prevents the stent from being placed in close proximity with the surface of the balloon. In contrast, metallic stents exhibit high plastic deformation and can be crimped closely to the balloon. This results in a lower profile that is more easily passed through the vasculature. One solution for decreasing the profile of a polymeric stent is to heat the stent so that the crimped profile will be retained. One potential drawback to this approach, however, is that the heat can cause permanent deformation that will not allow the polymeric stent to expand to the desired size and shape or heating can damage the stent lattice. Finally, during storage, the polymeric stent will undergo creep deformation making it difficult to expand the stent without applying heat to the stent at the deployment location.

WO-03/092550A1 discusses stent balloon assembly methods. The balloon is blow moulded inside the stent. Segments of the balloon at least partially fill gaps in the stent, even after the stent is compressed, to retain the stent on the balloon during delivery. To mount the stent balloon assembly on a catheter it is bonded to a catheter shaft and then crimped to plastically deform the stent into a compressed configuration for delivery.

US-6063092 relates to a heat set and crimping process to optimize stent retention. An expandable stent is crimped to a balloon. The resulting assembly is then constrained by a tube and shrink wrap. This prevents expansion of the stent when the balloon is pressurized. The assembly is heated to embed the stent in the balloon surface.

In view of the above, a need exists for an apparatus and method that can more reliably and accurately emplace a medical device such as a stent within a vessel or passageway of a patient.

The method of the present invention overcomes the limitations as briefly described above.

According to the present invention, there is provided a method as defined in appended claim 1. An expandable medical device is retained onto an expansion member so as to ensure reliable and accurate placement of the device within a vessel or passageway is provided. In particular, an expandable polymeric device such as a polymeric stent is mounted to an expansion member so as to prevent slippage of the polymeric stent relative to the expansion member during delivery while also exhibiting a low profile allowing for passage through the vasculature.

A polymeric stent having a latticed structure is placed on an expansion member and a constraining member such as a tube is then placed around the stent. The constraining member may be constructed from PTFE or any other inert material exhibiting a low coefficient of friction. The constraining member retains the stent in proximity to the expansion member. The constraining tube having the expansion member and stent therein is placed in a heating element. The expansion member is heated and an inert gas is placed in fluid communication with the expansion member at a pressure between 0.34 MPa (50 psi) and 2.41 MPa (350 psi). The expansion member partially inflates and nests within the lattice of the stent that has been softened by the heating process.

Once the supply of inert gas is discontinued and the constraining member removed from the heating block the expansion member having the polymeric stent mounted thereon is allowed to cool within the constraining member. When removed from the nesting tube the expansion member protrudes through, and is bonded to, the stent lattice. For example, a portion of the expansion member will protrude from between struts, flexible connectors, and curved joining members. This crimping arrangement ensures that the stent does not slip during navigation through the vasculature.

Embodiments of the invention will now be described by way of example only with reference to the accompanying drawings, in which:
Figure 1 is a perspective view of a delivery system for a stent showing the stent mounted to a distal end thereof.
Figure 2 is a longitudinal cross-section view of a delivery system.
Figure 3 is a perspective view of a polymeric stent to be mounted on an expansion member of a delivery system pursuant to the method of the present invention.
Figure 4 is a schematic view showing a stent placed on an expansion member to be inserted into a tubular member.
Figure 5 is a schematic view showing the tubular member having the expansion member and stent therein and placed into a heating element.
Figure 6 is a side view showing the stent of Figure 1 mounted onto the expansion member such that the expansion member protrudes through the lattice of the stent.
Figure 7 is a graphical representation showing the correlation between stent retention and the pressure applied to the expansion member during the method of the present invention.
Figure 8 is a perspective view of a balloon contoured in accordance with a method of the present invention.
Figure 9 is a schematic view showing the tubular member having a contoured expansion member and stent therein both placed into a heating element
Figure 10 is a graphical representation of stent retention for contoured v. non-contoured expansion members prepared in accordance with the method of the present invention.

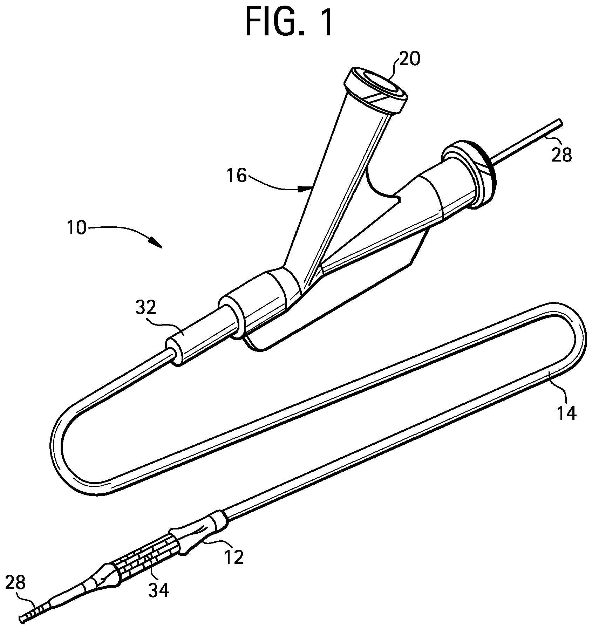

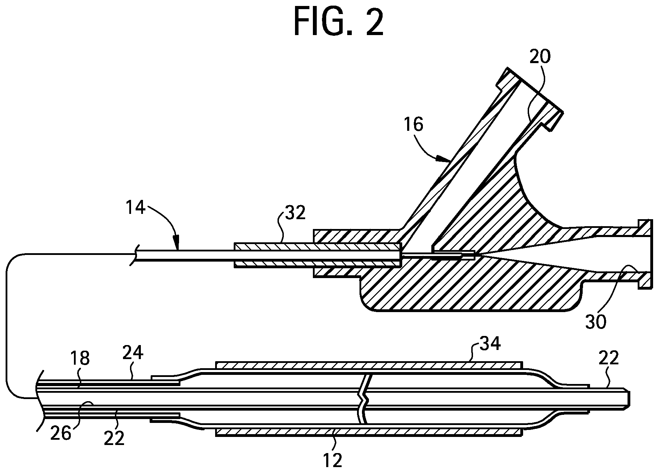

An example of a delivery system 10 for a medical device is shown in Figures 1-2. The delivery system 10 has an expansion member 12, a relatively long and flexible tubular shaft 14, and a hub 16. The expansion member 12 is affixed to the shaft 14 near a distal end thereof, and the hub 16 is affixed to the proximal end of the shaft 14. The shaft 14 defines one or more passages or lumens extending through the shaft, at least one of which is an inflation lumen 18 in fluid communication with the expansion member 12 in order to selectively expand and contract member 12. A hub inflation port 20 allows for an inflation source to communicate with the lumen 18.

In the illustrated embodiment, the shaft 14 comprises an inner and outer body 22 and 24. The inner body 22 defines a guidewire lumen 26, while the inflation lumen 18 is defined by the annular space between the inner and outer bodies 22 and 24. The guidewire lumen 26 is adapted to receive an elongated flexible guidewire 28 in a sliding fashion, such that the guidewire 28 and device 10 may be guided along a path defined by the guidewire 28. For example, guidewire 28 is typically positioned within a passageway or vessel prior to insertion of device 10 therein. The shaft 14 may have various configurations instead of a coaxial design, including a single extruded tube defining any suitable number of parallel side-by-side lumens, or a proximal shaft portion formed of a metal hypotube connected to a polymer distal shaft portion or other designs. Moreover, the catheter shaft may have a rapid exchange configuration, in which the guidewire exits the shaft at a proximal guidewire port located between the balloon and the hub.

The proximal hub 16 is affixed to the proximal end of the shaft 14, and preferably provides an inflation port 20 and a guidewire port 30, again with a luer-lock fitting or hemostatic valve (not shown in the figures). Such a valve allows the guidewire 28 to traverse and slide within the lumen 26, yet resist the loss of blood or other fluids through the guidewire lumen 26 or port 30.

As shown in the drawings, the inner and outer bodies 22 and 24 are securely received within the hub 16, and surrounded by a tubular strain relief 32. The hub 16 provides a fluid communication between the guidewire lumen 26 and a guidewire port 30 as well as between the inflation lumen 18 and the inflation port 20 and coupling.

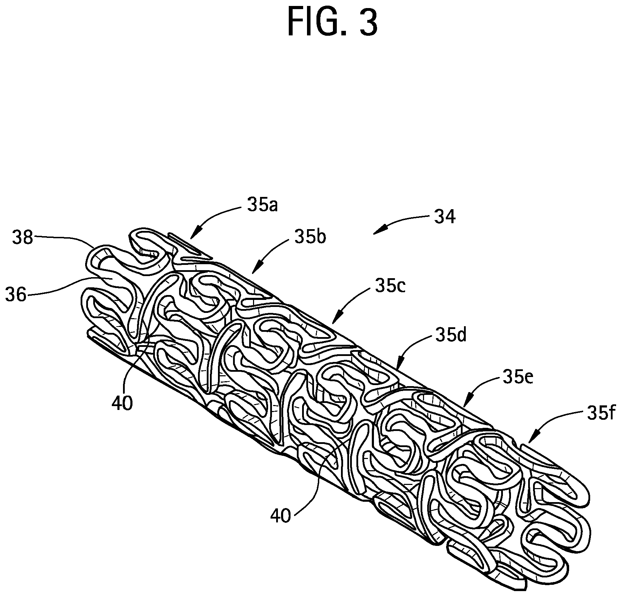

A polymeric stent 34 is shown in Figure 3. Stent 34 may be affixed to the expansion member 12 of the system 10 for delivery and deployment at a targeted site within the vasculature of the patient. Stent 34 shown in Figure 3 is generally cylindrical having a crimped diameter and a deployed diameter that is obtained when member 12 having the stent 34 mounted thereon is expanded. The stent 34 generally comprises a lattice made up of a series of loops 35a-f. Each loop 35 comprises a series of longitudinal struts 36 joined together by generally semi-circular joining members 38. Each adjacent loop 35a-e is connected together by flexible links 40. The loops 35, struts 36, members 38 and flexible links 40 can be varied in thickness, number and location to change the mechanical characteristics of the stent 34. For example, the point of attachment of flexible links 40 varies from one loop 35a-f to the next providing added flexibility for the stent 34.

Stent 34 is constructed from bioabsorbable polymeric materials. The stent 34 may comprise bioabsorbable or biostable polymers with drugs or other bio-active agents and radiopaque markers incorporated therein. Drugs or other bio-active agents may be incorporated into or coated onto the stent 34 in commonly used amounts or significantly greater amounts. Likewise, radiopaque markers may be provided in or on the stent 34. The combination of greater amounts of drugs or other agents for delivery from the device and the radiopaque markers improves the treatment of the targeted site, disease or condition and improves the visualization and placement of the device in the patient by the medical practitioner. The bioabsorbable polymeric materials that comprise the stent 34 or other device according to the systems and methods of the invention are chosen based on several factors, including degradation time, retention of the mechanical properties of the stent 34 or other device during the active drug delivery phase of the device, and the ability of the bioabsorbable materials to be processed into different structures and via different methods. Other factors, including cost and availability, may also be considered. Bioabsorbable polymeric materials that comprise the stent 34 may include shape memory polymers, polymer blends and/or composites that contribute to retaining the mechanical integrity until drug delivery is completed.

Examples of bulk erosion polymers employed for the manufacture of stent 34 include poly (α-hydroxy esters) such as poly (lactic acid), poly (glycolic acid), poly (caprolactone), poly (p-dioxanone), poly (trimethylene carbonate), poly (oxaesters), poly (oxaamides), and their co-polymers and blends. Some commercially readily available bulk erosion polymers and their commonly associated medical applications include poly (dioxanone) [PDS suture], poly {glycolide) [Dexon suture], poly (lactide)-PLLA [bone repair], poly (lactide/glycolide) [Vicryl (10/90) and Panacryl (95/5) sutures], poly (glycolide/caprolactone (75/25) [Monocryl suture], and poly (glycolide/trimethylene carbonate) [Maxon suture].

Other bulk erosion polymers employed are tyrosine derived poly amino acid [examples: poly (DTH carbonates), poly (arylates), and poly (imino-carbonates)], phosphorous containing polymers [examples: poly (phosphoesters) and poly (phosphazenes)], poly (ethylene glycol) [PEG] based block co-polymers [PEG-PLA, PEG-poly (propylene glycol), PEG-poly (butylene terphthalate)], poly (α -malic acid), poly (ester amide), and polyalkanoates [examples: poly (hydroxybutyrate (HB) and poly (hydroxyvalerate) (HV) co-polymers]. Other surface erosion polymers include poly (anhydrides) and poly (ortho esters).

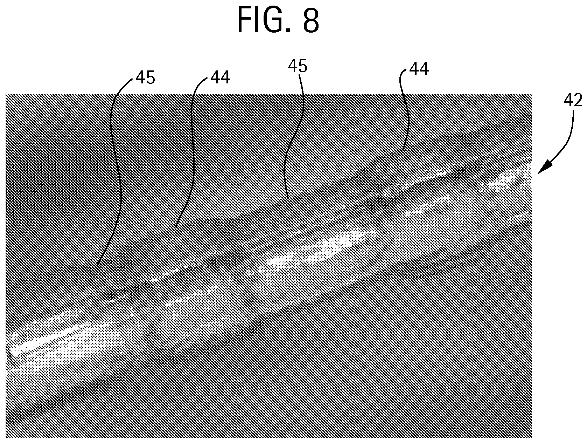

The plastic deformation of metallic stents allows for tight crimping around expansion member 12 so as to provide a low profile and prevent slippage as the stent 34 is navigated through the vasculature of a patient. Even when a metallic stent is crimped closely to the expansion member 12, however, slippage can occur. In order to further prevent slippage, expansion members can be modified to cooperate with the geometry of the stents. For example, as shown in Figure 8, a balloon 42 can be formed having contours 44. The contours 44 can extend through the lattice of a stent when mounted on balloon 42. Of course, this requires that the geometric features of the stent 34 be precisely aligned with the contours 44 of the balloon during crimping. This can prove somewhat difficult with misalignment leading to slippage or improper inflation of the stent.

It is more difficult to mount polymeric stent 34 onto an expansion member 12. While balloon expandable metallic stents are plastic in nature, polymeric stent 34 is somewhat elastic. This prevents the stent 34 from being tightly crimped onto the balloon unless elastic recovery is compensated for by, for example heating stent 34. This can lead, however, to permanent deformation that may adversely impact the structural integrity of polymeric stent 34.

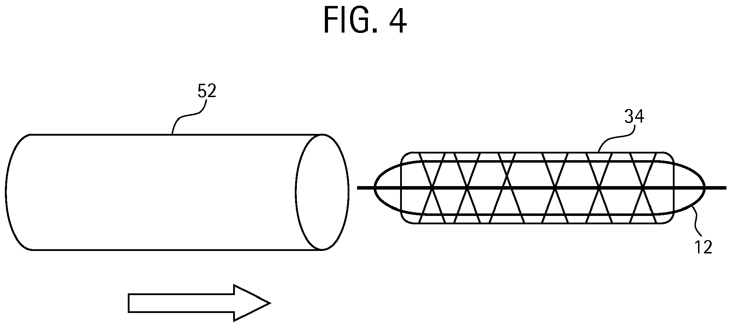

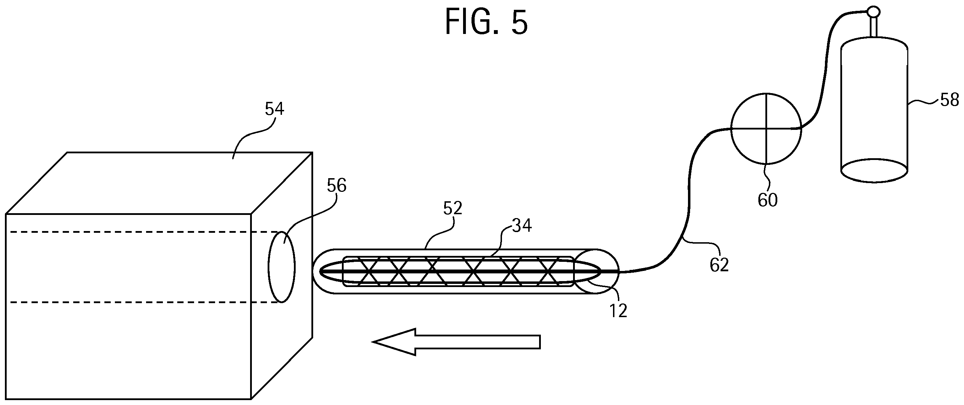

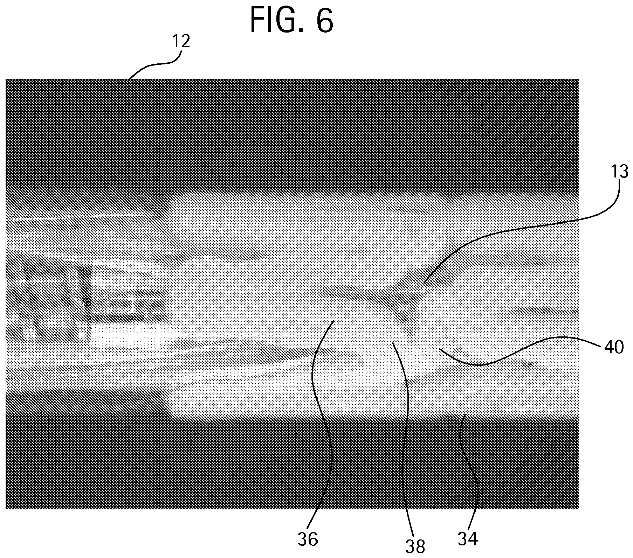

According to a method of the present invention a polymeric stent 34 is mounted to an expansion member 12. As shown in Figure 4, a nesting tube 52 was placed on the stent 34. The nesting tube 52 can be constructed from PTFE or any other inert material exhibiting a low coefficient of friction. The nesting tube constrains the stent 34 onto the expansion member 12. As shown in Figure 5, the tube 52 having expansion member 12 and stent 34 therein is placed in slot 56 located within a heating block 54. After the tube 52 is inserted into the slot 56, the expansion12 is heated. An inert gas source 58 via supply line 62 is placed in fluid communication with inflation port 20 and expansion member 12 is partially inflated. A pressure regulator 60 allows for the inflation pressure to be precisely controlled. The expansion member 12 expands and nests within the lattice of stent 34 that has been softened by the heating process. Thereafter, the supply of inert gas is discontinued and nesting tube 52 is removed from the heating block 54. Expansion member 12 having stent 34 mounted thereon is allowed to cool within the nesting tube 52. As shown in Figure 6, when removed from the nesting tube 52 the expansion member 12 protrudes from, and is bonded to, the stent lattice. For example, portion 13 of expansion member 12 protrudes from between the struts 36, flexible connectors 40 and curved joining members 38 of stent 34. This crimping arrangement ensures that stent 34 does not slip during navigation through the vasculature.

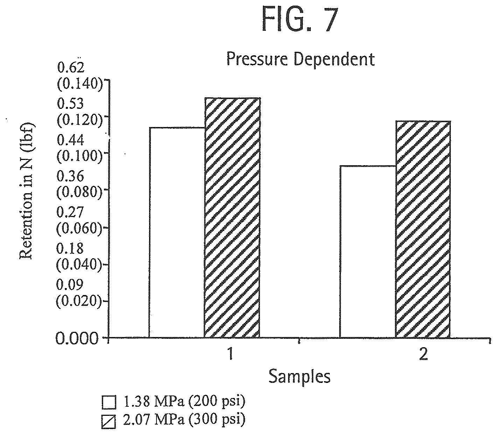

Nesting conditions depended on the type of material (amorphous, crystalline, etc) used to prepare the stent 34. In addition, other variables for nesting the stents were temperature of the heating block 54, time within heating block 54 and the pressure of the inert gas used to inflate expansion member 12 during nesting. Figure 7 illustrates the effect of pressure of the inert gas supplied to the expansion member 12 during the nesting process on stent retention. Although the variables can be adjusted, it is desirable that the stent 34 does not become physically deformed or melt during the nesting process. Typical nesting conditions were 50 to 100°C at 1.38 to 2.07 MPa (200 to 300 psi) of inflation pressure for 1 to 5 minutes. The preferred conditions were 50 to 60°C; 1.38 MPa (200 psi) and 2 to 5 minutes. This resulted in stent retention values of about 0.9 to 2.7 N (0.2 to 0.6 pounds).

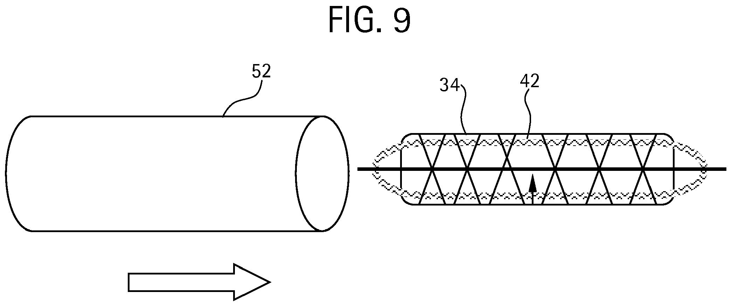

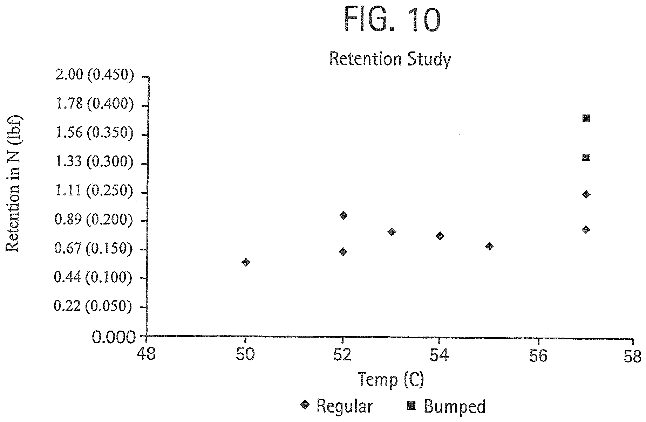

The method of the present invention may be employed with an expansion member 42 shown in Figure 8. In addition, expansion members can be treated to change the surface roughness or tackiness to improve retention. For example, materials such as PVP and PEG can be coated on the balloon to improve the stent adhesion to the balloon. As shown in Figure 9, the contoured balloon 42 of Figure 8 has stent 34 mounted thereon. It is desirable to match the contours 44 of the expansion member 12 to the openings in the lattice of stent 34. If this is not obtainable, however, the method of the present invention may still be carried out and significant retention is exhibited. For example, inflation of the balloon 42 will allow the non-contoured regions 45 to expand into the lattice of stent 34. Figure 10 illustrates the effects of varying temperature on stent retention during nesting using a bumped expansion member and a regular expansion member. Stents were nested in a range of 50°C to 60°C ±0.2°C with a pressure of 1.38 MPa (200psi) to 2.07 MPa (300psi) ± 0.07 MPa (10psi) for 2min to 5min ±5sec.

Although shown and described is what is believed to be the most practical and preferred embodiments, it is apparent that departures from specific designs and methods described and shown will suggest themselves to those skilled in the art and may be used without departing from the scope of the invention. The present invention is not restricted to the particular constructions described and illustrated, but should be constructed to cohere with all modifications that may fall within the scope for the appended claims.

## Claims

1. A method of retaining a polymeric stent (34) on an expansion member (12, 42) comprising the steps of:
placing a polymeric stent (34) on an expansion member (12, 42);
encasing the polymeric stent (34) and expansion member (12, 42) within a tubular member (52);
placing the tubular member (52) within a temperature controlled element (54);
heating the tubular member (52) within the temperature controlled element (54);
pressurizing the expansion member (12, 42) to a pressure between 0.34 MPa (50 psi) and 2.41 MPa (350 psi) whereby the expansion member (12, 42) extends through the polymeric stent (34) and contacts an inner surface of the tubular member (52);
depressurizing the expansion member (12, 42); and
removing the tubular member (52) from the temperature controlled element (54).

2. The method of claim 1 wherein the expansion member (12, 42) comprises a balloon mounted on a catheter having a flexible shaft with a proximal and a distal end.

3. The method of claim 1 wherein the tubular member (52) comprises a biocompatible inert material with low coefficient of friction.

4. The method of claim 1 wherein the polymeric stent (34) comprises a bioabsorbable polymer.

5. The method of claim 1 wherein the temperature of the temperature controlled element (54) ranges from 25°C to 150°C.

6. The method of claim 1 wherein the step of pressurizing the expansion member (12, 42) comprises raising the pressure of the expansion member (12, 42) between 1.38 MPa (200 psi) to 2.01 MPa (300 psi).

7. The method of claim 1 wherein the time for pressurizing the expansion member (12, 42) ranges from 30 seconds to 10 minutes.

8. The method of claim 1 wherein the retention force of the polymeric stent (34) on the expansion member (12, 42) ranges from 0.4 to 4.4 N (0.1 to 1 lbs).

9. The method of claim 1 wherein the expansion member (42) is contoured.

10. The method of claim 1 wherein the expansion member (42) has a modified surface.

## Patentansprüche

1. Verfahren zum Halten eines polymeren Stents (34) an einem Erweiterungselement (12, 42), umfassend die Schritte des:
Positionierens eines polymeren Stents (34) auf ein Erweiterungselement (12, 42);
Einschließens des polymeren Stents (34) und des Erweiterungselements (12, 42) innerhalb eines röhrenförmigen Elements (52);
Positionierens des röhrenförmigen Elements (52) innerhalb eines temperaturgeregelten Teils (54),
Erhitzens des röhrenförmigen Elements (52) innerhalb des temperaturgeregelten Teils (54);
Unterdrucksetzens des Erweiterungselements (12, 42) auf einen Druck zwischen 0,34 MPa (50 psi) und 2,41 MPa (350 psi), wodurch das Erweiterungselement (12, 42) sich durch den polymeren Stent (34) erstreckt und eine Innenfläche des röhrenförmigen Elements (52) kontaktiert;
Drucklosmachens des Erweiterungselements (12, 42); und
Entfernens des röhrenförmigen Elements (52) aus dem temperaturgeregelten Teil (54).

2. Verfahren nach Anspruch 1, wobei das Erweiterungselement (12, 42) einen Ballon umfasst, der auf einem Katheter montiert ist, der einen flexiblen Schaft mit einem proximalen und einem distalen Ende aufweist.

3. Verfahren nach Anspruch 1, wobei das röhrenförmige Element (52) ein biokompatibles inertes Material mit einem niedrigen Reibungskoeffizienten umfasst.

4. Verfahren nach Anspruch 1, wobei der polymere Stent (34) ein biologisch absorbierbares Polymer umfasst.

5. Verfahren nach Anspruch 1, wobei die Temperatur des temperaturgeregelten Teils (54) im Bereich von 25 °C bis 150 °C liegt.

6. Verfahren nach Anspruch 1, wobei der Schritt des Unterdrucksetzens des Erweiterungselements (12, 42) das Erhöhen des Drucks des Erweiterungselements (12, 42) auf 1,38 MPa (200 psi) bis 2,01 MPa (300 psi) umfasst.

7. Verfahren nach Anspruch 1, wobei die Zeit zum Unterdrucksetzen des Erweitungselements (12, 42) im Bereich zwischen 30 Sekunden und 10 Minuten liegt.

8. Verfahren nach Anspruch 1, wobei die Rückhaltekraft des polymeren Stents (34) auf dem Erweiterungselement (12, 42) im Bereich von 0,4 bis 4,4 N (0,1 bis 1 Pfund) liegt.

9. Verfahren nach Anspruch 1, wobei das Erweiterungselement (42) konturiert ist.

10. Verfahren nach Anspruch 1, wobei das Erweiterungselement (42) eine modifizierte Oberfläche aufweist.

## Revendications

1. Procédé de maintien d'une endoprothèse polymère (34) sur un élément d'expansion (12, 42) comprenant les étapes de:
mise en place d'une endoprothèse polymère (34) sur un élément d'expansion (12, 42);
emboîtage de l'endoprothèse polymère (34) et de l'élément d'expansion (12, 42) à l'intérieur d'un élément tubulaire (52);
mise en place de l'élément tubulaire (52) à l'intérieur d'une partie à température contrôlée (54);
chauffage de l'élément tubulaire (52) à l'intérieur de la partie à température contrôlée (54);
mise sous pression de l'élément d'expansion (12, 42) jusqu'à une pression comprise entre 0,34 MPa (50 lb/po²) et 2,41 MPa (350 lb/po²) moyennant quoi l'élément d'expansion (12, 42) s'étend à travers l'endoprothèse polymère (34) et entre en contact avec une surface interne de l'élément tubulaire (52);
dépressurisation de l'élément d'expansion (12, 42); et
retrait de l'élément tubulaire (52) de la partie à température contrôlée (54).

2. Procédé selon la revendication 1, dans lequel l'élément d'expansion (12, 42) comprend un ballonnet monté sur un cathéter présentant une tige flexible avec une extrémité proximale et une extrémité distale.

3. Procédé selon la revendication 1, dans lequel l'élément tubulaire (52) comprend un matériau inerte biocompatible à un faible coefficient de friction.

4. Procédé selon la revendication 1, dans lequel l'endoprothèse polymère (34) comprend un polymère bioabsorbable.

5. Procédé selon la revendication 1, dans lequel la température de la partie à température contrôlée (54) s'étend de 25°C à 150°C.

6. Procédé selon la revendication 1, dans lequel l'étape de pressurisation de l'élément d'expansion (12, 42) comprend l'élévation de la pression de l'élément d'expansion (12, 42) entre 1,38 MPa (200 lb/po²) à 2,01 MPa (300 lb/po²).

7. Procédé selon la revendication 1, dans lequel le temps de pressurisation de l'élément d'expansion (12, 42) s'étend de 30 secondes à 10 minutes.

8. Procédé selon la revendication 1, dans lequel la force de rétention de l'endoprothèse polymère (34) sur l'élément d'expansion (12, 42) s'étend de 0,4 à 4,4 N (0,1 à 1 lb).

9. Procédé selon la revendication 1, dans lequel l'élément d'expansion (42) est profilé.

10. Procédé selon la revendication 1, dans lequel l'élément d'expansion (42) présente une surface modifiée.
